# FASCICULE DE BREVET EUROPEEN

(11) **EP 0 489 269 B1**
(45) Date de publication et mention de la délivrance du brevet: **13.09.1995**
(21) Numéro de dépôt: 91119036.1
(22) Date de dépôt: 08.11.1991
(51) Int. Cl.: B65D 75/32, B65D 81/24

(54) **Emballage en matière plastique à compartiments multiples pour produits liquides et solides**
Verpackung aus Kunststoff mit mehreren Kammern für flüssige und feste Produkte
Plastic package with multiple compartments for liquid and solid products

(30) Priorité: 05.12.1990 CH 3846/90
(43) Date de publication de la demande: 10.06.1992
(73) Titulaire: FIRMENICH SA, CH-1211 Genève 8 (CH)
(72) Inventeur: Holzner, Günter, CH-1212 Grand-Lancy (CH)
(74) Mandataire: Salvadori, Giuseppe

(56) Documents cités:
- WO-A-82/02700
- DE-A- 2 201 305
- FR-A- 2 539 492
- US-A- 4 161 283

## Description

L'invention concerne le domaine de l'emballage et du conditionnement. Elle a en particulier pour objet un emballage industriel, bon marché et prêt à l'emploi (dose unité) destiné à contenir des dispositifs pour le parfumage d'air ambiant ou d'enceintes fermées.

La demande internationale WO 81/00051, publiée le 22 janvier 1981, décrit un article étanche aux liquides destiné au parfumage d'air ambiant ou d'enceintes fermées, caractérisé en ce qu'il contient une composition parfumante enrichie en substances, odorantes ou pas, de faible polarité et qu'il possède au moins une paroi polymérique permettant la diffusion vers l'extérieur d'ingrédients odoriférants actifs.

La demande de brevet française FR-A 2091855, publiée le 21 janvier 1972, concerne un dispositif pour la diffusion continue de vapeurs d'agents assainissants, qui comprend une paroi de perméation en matière macromoléculaire dont une des faces est en contact avec l'agent assainissant volatil, lequel agent imprègne une masse fibreuse qui est recouverte par la paroi de perméation.

La demande de brevet FR-A 2336946, publiée le 29 juillet 1977, concerne un système de rafraîchissement de l'air à diffusion lente, qui comprend une enceinte et un parfum contenu dans ladite enceinte, au moins une surface de ladite enceinte étant une pellicule d'un polymère. Dans ce système également, le parfum est imprégné sur un support tel que silice, talc, laine de verte ou papier buvard.

Le brevet US 3,578,545, publié le 11 mai 1971, concerne un stratifié flexible libérant un parfum qui comprend un support de tissu imprégné de parfum, entouré d'un film plastique poreux, perméable aux vapeurs de parfums.

Le brevet US 4,161,283, publié le 17 juillet 1979, décrit un dispositif destiné à diffuser de façon continue des vapeurs assainissantes; ledit dispositif comprend une paroi extérieure constituée par du matériel polymérique permettant une diffusion moléculaire et une paroi intérieure imperméable. La paroi extérieure est en outre recouverte d'une feuille imperméable apte à éviter la diffusion des substances volatiles actives lors du stockage.

Un dispositif analogue est décrit dans le brevet US 4,285,468, publié le 25 août 1981.

Les articles assainissants décrits dans l'art antérieur, étant tous constitués par un système comportant une paroi de diffusion polymérique, doivent posséder un dispositif destiné à empêcher la diffusion des éléments parfumants actifs dans l'environnement pendant le stockage.

A cet effet, la demande internationale WO 81/00051 décrit un moyen qui consiste en l'application d'une feuille polymérique du type Surlyn ® (origine : Du Pont de Nemours) sur les parois au travers desquelles s'effectue normalement la diffusion. Une telle feuille polymérique peut adhérer par soudage thermique à du polyéthylène ou du polypropylène et être détachée juste avant l'utilisation. Dans le même but, un autre moyen consiste en l'utilisation de feuilles d'aluminium soudées à de la mousse de polypropylène.

Un problème se pose, cependant, lors du stockage prolongé de tels dispositifs avant la vente : du fait de la diffusion des vapeurs de parfum au travers des parois en matière polymérique de l'emballage, on a pu constater une perte notable de la quantité initiale de substance active. Afin de pallier à cet inconvénient, un dispositif à chambres multiples a été développé par la demanderesse [voir demande de brevet internationale WO 82/02700, publiée le 19 août 1982]. Dans un tel dispositif, la solution parfumante active est conservée dans un compartiment aux parois imperméables aux vapeurs de parfum et dont l'un des joints peut être rompu sous l'effet d'une pression externe puis, juste avant usage, les parois dudit joint une fois rompues, la solution passe dans un compartiment aux parois en matière polymérique perméable aux vapeurs de parfums qui peuvent alors diffuser uniformément dans l'atmosphère ambiante.

A l'usage, un tel dispositif se montre très efficace, mais sa manufacture nécessite la mise au point d'un appareil perfectionné et une technique d'assemblage rigoureuse.

Comme remède à de tels inconvénients, la demande de brevet FR-A 2539492 propose une solution nouvelle basée sur une technique simple. Elle décrit en effet un assemblage direct par soudure d'une mince couche de papier sur la paroi externe de la membrane polymérique au travers de laquelle s'effectue la diffusion des vapeurs de substance active. Cette fine couche de papier est ensuite revêtue d'une feuille de matière polymérique, par exemple du type Surlyn ® à base de polyéthylène, sur laquelle est enfin soudée une feuille métallique, de préférence par exemple une feuille d'aluminium.

La présente invention apporte une solution nouvelle au problème posé par l'emballage de dispositifs perméables à des substances volatiles. Elle a en effet pour objet un emballage hermétique sous vide en matière plastique et à parois élastiques contenant des dispositifs pour le parfumage ou l'assainissement d'air ambiant ou d'enceintes fermées, lesdits dispositifs étant dotés d'une paroi en matière polymérique permettant la diffusion dans le milieu environnant d'une substance odoriférante ou désodorisante active, lequel emballage est caractérisé en ce qu'il contient au moins deux dispositifs, lesquels sont accolés en joignant par superposition, sous pression subatmosphérique, les surfaces de leurs parois polymériques de diffusion, laquelle pression est appliquée à l'intérieur de l'emballage contenant lesdits dispositifs lors de la soudure de ses parois élastiques, de façon que ces dispositifs restent joints à l'intérieur de l'emballage par le contact direct et intégral de leurs parois de diffusion.

L'emballage décrit est de réalisation particulièrement simple et peut être fabriqué à l'aide d'une installation industrielle courante. Par son emploi, la solution active peut être conservée sans perte aucune pendant des périodes prolongées avant l'usage. Au moment désiré, l'utilisateur pourra par contre aisément déchirer partiellement ou totalement l'emballage, en extraire les dispositifs et activer ainsi la diffusion des vapeurs de la substance active vers l'extérieur. En effet, lors de l'ouverture de l'emballage, il se produit un équilibrage de sa pression interne et de la pression environnante, ce qui provoque une séparation simultanée des dispositifs, accolés à l'origine, dont les parois de diffusion respectives se trouvent de ce fait exposées à l'air. Le phénomène de diffusion peut ainsi commencer.

### Description des dessins

Des formes d'exécution de l'emballage de l'invention seront décrites, à titre d'exemple, en se référant aux dessins annexés dans lesquels :
la **fig. 1** représente une vue d'un emballage comportant deux dispositifs de diffusion, avec sa coupe transversale et son éclatement longitudinal;
la **fig. 2** représente une vue d'un emballage comportant trois dispositifs de diffusion, avec sa coupe transversale et son éclatement longitudinal;
la **fig. 3** représente une vue d'un emballage comportant une série de dispositifs de diffusion appariés, avec sa coupe transversale et son éclatement longitudinal;
la **fig. 4** représente une vue en perspective de deux dispositifs de diffusion soudés par la partie supérieure de leur paroi.

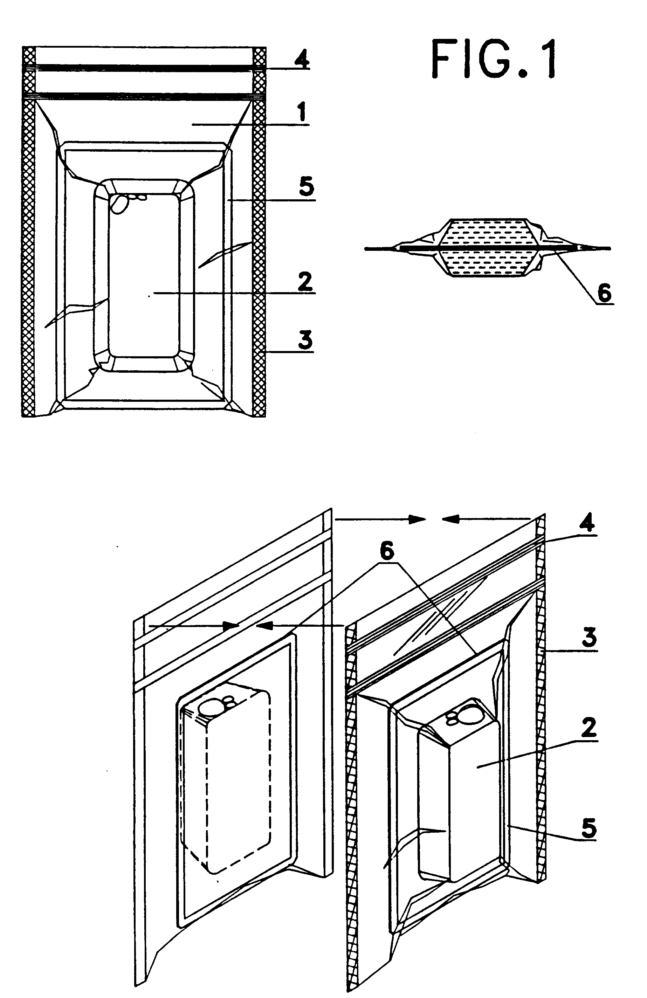

L'emballage 1 représenté à la fig. 1 se compose de deux dispositifs appariés 2, accolés en joignant par superposition les surfaces de leurs parois polymériques 6. Chaque dispositif comporte un bord 5 créé par la soudure de la paroi polymérique de diffusion avec une coque thermoformée. Les deux dispositifs sont maintenus dans un sachet hermétique formé par l'assemblage de deux feuilles polymériques soudées en leur pourtour de manière à former des joints déchirables 3 et 4.
Dans la représentation donnée à la fig. 1, les deux dispositifs sont remplis par une substance désodorisante ou assainissante liquide.

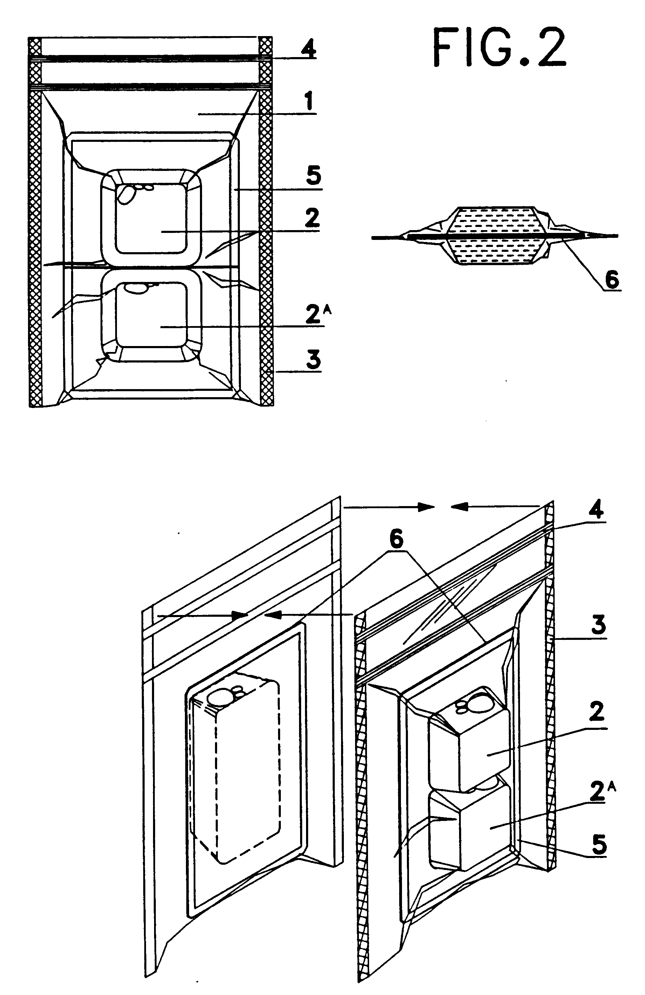

La fig. 2 représente une forme d'exécution particulière de l'emballage selon l'invention. Les dispositifs 2 contenant la substance active sont au nombre de trois Les deux dispositifs 2 et 2A sont assemblés en série et leur contenance est inférieure à la contenance du dispositif auquel ils sont accolés. Les deux dispositifs de volume inférieur peuvent être séparés par l'utilisateur grâce à une une médiane de soudure ou d'indentation.

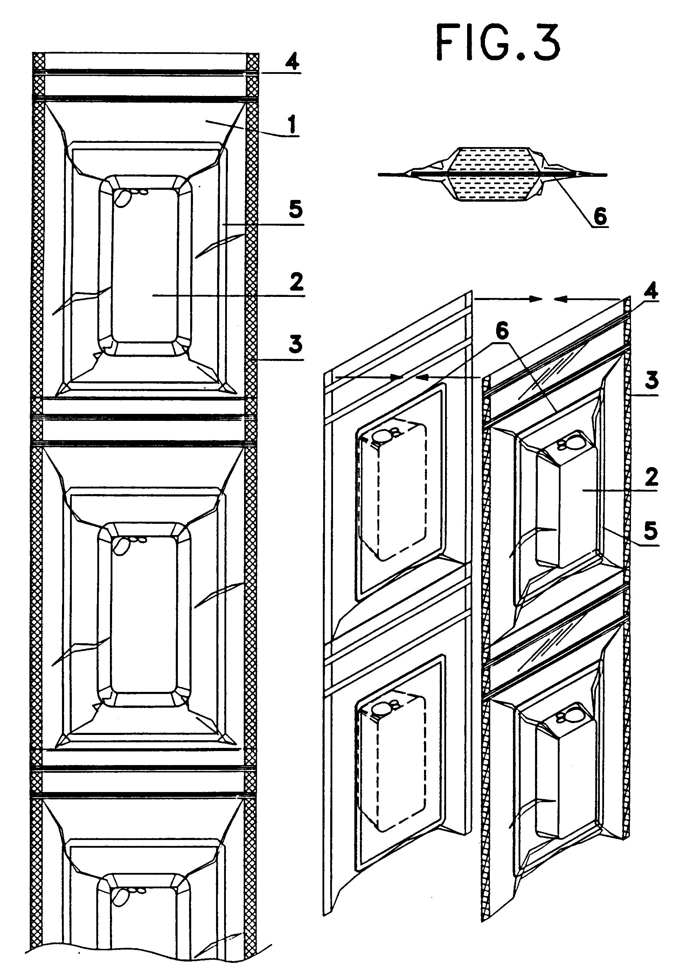

La fig. 3 représente un emballage 1 comportant une série de dispositifs 2 appariés dont la forme d'exécution est analogue à celle décrite à la fig. 1. De tels dispositifs peuvent être activés en déchirant l'emballage le long des bords de soudure 3 et 4 ou par coupure transversale.

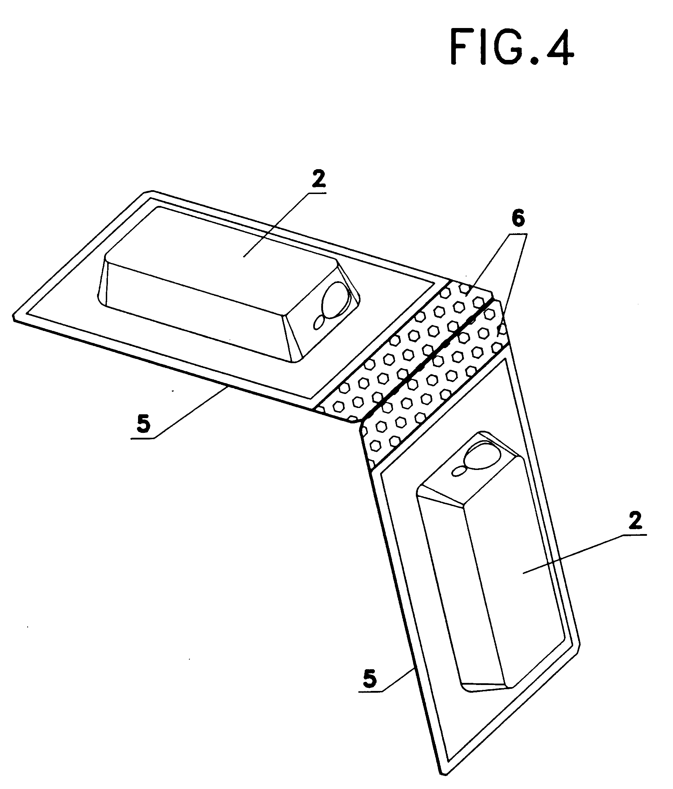

La fig. 4 représente une disposition particulière de deux dispositifs de diffusion appariés 2, soudés l'un à l'autre par le bord supérieur de leurs parois polymériques 6 avant d'être placés dans le sachet hermétique.
L'activation des dispositifs a lieu par exposition à l'air des parois polymériques de diffusion, ce qui s'effectue lors de l'ouverture de l'emballage et séparation de deux parties accolées, ainsi qu'il est montré par la vue éclatée selon un axe longitudinal donnée aux figs 1 à 3.

Pour d'autres formes d'exécution, on peut se reporter aux dispositifs particuliers décrits dans l'art antérieur.

L'avantage majeur présenté par l'emballage de l'invention réside non seulement dans la simplicité de son assemblage mais aussi dans le fait que l'activation s'effectue de manière particulièrement nette. Comme les dispositifs sont réunis par nombre de deux ou plus, il est entendu que l'activation a lieu simultanément sur deux dispositifs au moins. Ces deux dispositifs peuvent être employés soit conjointement pour le parfumage d'un même local ou enceinte fermée, soit séparément. Il demeure entendu que la forme particulière de ces dispositifs, et tout spécialement leur contenance, peut varier en fonction de l'emploi envisagé. Un aspect critique de l'invention demeure toutefois le fait que de tels dispositifs ont une paroi polymérique de diffusion à surface plane ou essentiellement plane de sorte que leur superposition, sous l'action du vide, puisse permettre une adhésion temporaire aussi parfaite que possible et ce jusqu'à l'ouverture de l'emballage.

Pour la réalisation de tels dispositifs, il est fait référence ici en particulier aux documents de l'art antérieur mentionnés plus haut. La paroi de diffusion peut donc être constituée par des feuilles de matériaux polymériques variés tels ceux décrits dans la demande de brevet internationale WO 81/00051.

Dans la mesure où l'adhésion des dispositifs à l'intérieur de l'emballage a lieu par un phénomène purement physique sans avoir recours à l'emploi d'adhésifs, la diffusion des vapeurs de la substance active n'est nullement entravée. Dans son exécution la plus simple, l'emballage de l'invention renferme deux seuls dispositifs ou réservoirs contenant la substance active. Il demeure entendu cependant qu'un tel emballage peut contenu un nombre non déterminé de dispositifs, la seule condition étant que ces dispositifs soient joints par le contact direct et intégral de leurs parois de diffusion respectives. Ainsi, un tel emballage pourra contenir un nombre pair de dispositifs identiques, les dispositifs étant alors appariés. Dans le cas où l'emballage contiendrait des dispositifs dont les surfaces de leurs parois de diffusion ne seraient pas identiques, il faudra veiller à assembler l'emballage de sorte que chaque dispositif "A" soit accolé à un nombre de dispositifs n x "B" (n étant un nombre entier) dont la somme des surfaces de leurs parois de diffusion soit égale à la surface de la paroi de diffusion de "A". Toute diffusion prématurée, avant l'activation par l'utilisateur, des vapeurs de la substance active est ainsi supprimée.

La fermeture de l'emballage s'effectue de préférence par simple soudure des extrémités des feuilles polymériques qui constituent ses parois. Elle a lieu sous vide, en appliquant donc une pression subatmosphérique, par exemple de l'ordre de quelques millimètres de mercure. Il s'agit d'une technique fort courante, largement appliquée à l'emballage par exemple d'aliments pour lesquels on veut garantir une longue durée de conservation, notamment ceux qui sont ensuite soumis à congélation. Les températures de soudage peuvent généralement varier entre 150 et 220°. Elles sont fonction bien entendu de la nature du matériau polymérique employé. Afin de faciliter son ouverture, l'emballage peut comporter des indentations ou échancrures à l'une des extrémités, voire un onglet grâce auquel l'utilisateur peut plus aisément séparer les feuilles constituant ses parois.

### Manufacture de l'emballage

On a procédé à la fabrication de dispositifs pour le parfumage ou l'assainissement d'air ambiant ou d'enceintes fermées de la manière suivante.
Une feuille composite de 250 µm de Barex (marque enregistrée de BP Chemicals; polyacrylonitrile) et 50 µm de polyéthylène a été thermoformée à l'aide d'un moule spécifique afin de lui conférer l'aspect et la dimension souhaités (appareil : Thermoforming Spa, Milan). La coque transparente obtenue a été ensuite assemblée par soudure à la membrane polymérique qui constitue la paroi de diffusion plane du dispositif en laissant toutefois une ouverture destinée au remplissage de la substance parfumante ou assainissante. Le volume de la chambre peut varier en fonction de l'emploi envisagé, en particulier en fonction de la durée d'action prévue. Il s'est révélé à l'usage que des dispositifs de contenance comprise entre 3 et 15cc pouvaient parfaitement convenir dans la plupart des cas considérés. La membrane est constituée de préférence par une feuille de copolymère consistant en du polyéthylène et polyéthylacrylate, par exemple 82:18.
L'appareil de soudure était du type Fermant 400 (Joisten & Kettenbaum GmbH, Bensberg-Herkenrath, Allemagne). Une fois terminé le remplissage de la chambre ainsi formée par la substance parfumante ou assainissante active, on procède à sa fermeture par soudure. Les dispositifs ainsi obtenus sont introduits dans un sachet, constitué par deux feuilles sandwich multicouches polymériques (ex.: polyamide et polyéthylène) soudées à trois de leurs extrémités, et accolés de sorte que leurs parois de diffusion respectives soient disposées face à face en opposition. Le sachet est ensuite introduit dans un appareil à souder équipé d'un système d'aspiration (ex.: type VC 999, Inauen Maschinen AG, Herisau, Suisse). On applique un vide égal à 1-2 mmHg et une température de 180-220°C, ce qui a pour résultat de provoquer la fermeture hermétique du sachet tout en provoquant un contact parfait des parois de diffusion des dispositifs.

## Revendications

1. Emballage (1) hermétique sous vide en matière plastique et à parois élastiques contenant des dispositifs (2,2A) pour le parfumage ou l'assainissement d'air ambiant ou d'enceintes fermées, lesdits dispositifs étant dotés d'une paroi (6) en matière polymérique permettant la diffusion dans le milieu environnant d'une substance odoriférante ou désodorisante active, lequel emballage (1) est caractérisé en ce qu'il contient au moins deux dispositifs (2,2A), lesquels sont accolés en joignant par superposition, sous pression subatmosphérique, les surfaces de leurs parois (6) polymériques de diffusion, laquelle pression est appliquée à l'intérieur de l'emballage (1) contenant lesdits dispositifs (2,2A) lors de la soudure de ses parois élastiques, de façon que ces dispositifs restent joins à l'intérieur de l'emballage (1) par le contact direct et intégral de leurs parois (6) de diffusion.

2. Emballage selon la revendication 1, contenant un nombre pair de dispositifs (2,2A) pour le parfumage ou l'assainissement d'air ambiant ou d'enceintes fermées et dans lequel lesdits dispositifs sont accolés par paire.

3. Emballage selon la revendication 2, contenant deux dispositifs (2,2A) pour le parfumage ou l'assainissement d'air ambiant ou d'enceintes fermées.

4. Emballage selon la revendication 1, caractérisé en ce que la paroi (6) polymérique de diffusion des dispositifs (2,2A) de parfumage ou d'assainissement est constitué par une membrane en matière polymérique simple ou composite recouverte d'une mince couche de papier.

5. Emballage selon la revendication 1, caractérisé en ce que l'activation des dispositifs (2,2A) s'effectue par ouverture de l'emballage (1), équilibrage de sa pression interne et de la pression environnante et séparation des dispositifs (2,2A) à l'origine accolés, ce qui entraîne une exposition à l'air de leurs parois (6) de diffusion et une lente évaporation de la substance parfumante ou assainissante active qu'ils renferment.

6. Emballage selon l'une des revendications précédentes, caractérisé en ce que ses parois élastiques sont constituées par des feuilles de matériau polymérique dérivé d'un polyester ou d'un polyamide, ou par des feuilles sandwich multicouches de l'un de ces deux polymères avec le polyéthylène.

## Claims

1. A plastic hermetically sealed package (1), under vacuum, having resilient walls, containing devices (2,2A) for perfuming or purifying ambient air or closed spaces, said devices having a polymeric wall (6) enabling the diffusion of an active odoriferous or deodorant substance into the surrounding environment, which package (1) is characterized in that it contains at least two such devices (2,2A), the latter being joined side by side by superposition, under subatmospheric pressure, of the surfaces of their polymeric diffusion walls (6), the pressure being applied inside the package (1) containing said devices (2,2A) when said resilient walls are sealed, such that these devices stay joined inside the package (1) through the direct and total contact of their diffusion walls (6).

2. A package according to claim 1, containing an even number of devices (2,2A) for perfuming or purifying ambient air or closed spaces and wherein said devices are joined pairwise.

3. A package according to claim 2, containing two devices (2,2A) for perfuming or purifying ambient air or closed spaces.

4. A package according to claim 1, characterized in that said polymeric diffusion wall (6) of said perfuming or purifying devices (2,2A) is formed of a simple or composite polymeric material covered by a thin paper layer.

5. A package according to claim 1, characterized in that activation of said devices (2,2A) takes place when the package (1) is opened, its internal pressure being balanced by the pressure of the surroundings, and the originally joined devices (2,2A) separate, their diffusion walls (6) being thus exposed to the air, such that slow evaporation of the perfuming or purifying substance contained in said devices results.

6. A package according to any one of the preceding claims, characterized in that said resilient walls are formed of sheets of polymeric material derived from a polyester or a polyamide, or of sandwich multilayer sheets of one of these polymers with polyethylene.

## Patentansprüche

1. Unter Vakuum stehende hermetische Verpackung (1) aus Kunststoff mit elastischen Wänden, die Vorrichtungen (2,2A) zum Parfümieren oder zur Verbesserung der umgebenden Luft oder der Luft von geschlossenen Räumen enthält, welche eine polymere Wand (6), die die Diffusion einer aktiven Duft- oder desodorisierenden Substanz in die Umgebung erlaubt, enthalten, dadurch gekennzeichnet, daß die Verpackung (1) wenigstens zwei Vorrichtungen (2,2A) enthält, zusammengefügt durch Überlagerung der polymeren Diffusionsoberflächen unter subatmosphären Druck, welcher im Inneren der Verpackung (1), die die Vorrichtungen (2,2A) enthält, während des Verschweißens der elastischen Wände, so angewendet wird, daß diese Vorrichtungen durch den direkten Vollkontakt ihrer Diffusionswände (6) im Innern der Verpackung (1) verbunden bleiben.

2. Verpackung gemäß Patentanspruch 1, die eine gerade Zahl der Vorrichtungen (2,2A) zum Parfümieren oder zur Verbesserung der umgebenden Luft oder der Luft von geschloßenen Räumen enthält und worin diese Vorrichtungen paarweise zusammengefügt sind.

3. Verpackung gemäß Patentanspruch 2, die zwei Vorrichtungen (2,2A) zum Parfümieren oder zur Verbesserung der umgebenden Luft oder der Luft von geschloßenen Räumen enthält.

4. Verpackung gemäß Patentanspruch 1, dadurch gekennzeichnet, daß die polymere Diffusionswand (6) der Vorrichtungen (2,2A) zum Parfümieren oder zur Verbesserung aus einer Membran besteht, die aus einem einfach oder mischpolymeren Material hergestellt und mit einer dünnen Papierschicht bedeckt ist.

5. Verpackung gemäß Patentanspruch 1, dadurch gekennzeichnet, daß die Vorrichtungen (2,2A) nach dem Öffnen der Verpackung (1), nach Gleichgewichtseinstellung ihres Innen- und Umgebungsdrucks und nach Trennung der anfangs zusammengefügten Vorrichtungen (2,2A), aktiviert werden, was zum Auslüften ihrer Diffusionswände (6) und zu einem langsamen Verdampfen der enthaltenen aktiven Duft- oder desodorisierenden Substanz führt.

6. Verpackung gemäß einem der vorhergehenden Patentansprüche, dadurch gekennzeichnet, daß ihre elastischen Wänden aus Polymerfolien bestehen, welche von einem Polyester- oder Polyamidderivat oder von einer Verbundfolien eines dieser zwei Polymeren mit Polyäthylen, abgeleitet sind.
